# EUROPEAN PATENT APPLICATION

(11) **EP 4 607 225 A1**
(43) Date of publication of application: **27.08.2025**
(21) Application number: 24159684.0
(22) Date of filing: 26.02.2024
(51) Int. Cl.: G01R 33/44, G01R 33/3815, G01R 33/38, A61B 5/055, A61B 5/00

(54) **TILTABLE SUPERCONDUCTING MAGNETIC RESONANCE IMAGING SYSTEM**

(71) Applicant: Koninklijke Philips N.V., 5656 AG Eindhoven (NL)
(72) Inventor: LIPS, Oliver, Eindhoven (NL); FORTHMANN, Peter, Eindhoven (NL); VERNICKEL, Peter, 5656AG Eindhoven (NL)
(74) Representative: Philips Intellectual Property & Standards

(57) **Abstract**

A magnetic resonance imaging system is presented, including: a magnetic resonance imager (302) comprising a superconducting magnet (304) surrounding a bore (306); a cooling system configured to cool the superconducting magnet through conduction cooling; a frame (520) rotatably supporting the magnetic resonance imager; an actuator configured to move the magnetic resonance imager in a plurality of predetermined examination positions between a horizontal orientation and a vertical orientation of a main static magnetic field associated to the superconducting magnet; and a patient support (320) configured to support a patient in any of the plurality of predetermined examination positions.

## Description

### TECHNICAL FIELD

The subject matter described herein relates to magnetic resonance imaging (MRI) systems, in particular based on superconducting magnets.

### BACKGROUND

Weight bearing magnetic resonance imaging (WBMRI) enables examinations of a patient in vertical position for assessment of the spine and joints. In finding the root cause of various pathologies, e.g., lower back pain, it is necessary to account for the force of gravity during examination, which generates bio-mechanical changes in the anatomy.
Clinical applications that particularly benefit from the invention are assessment of:
stability or instability of the spine, e.g. olisthesis, scoliosis;
degenerative pathologies in spine, e.g., protrusion, herniation;
spinal stenosis, e.g., ligament and facet joints; postoperative assessment of re-herniation.

WBMRI is currently only feasible in specialized systems, which typically employ heavy iron yokes or permanent magnets and work at lower field strengths smaller than 1 Tesla (T). One such commercially available system is the G-scan Brio of Esaote S.p.A. utilizing an open permanent magnet, rotating gantry and passive shimming, operating at magnetic field strength of 0.25 T. The state-of-the-art MRI systems having superconductive magnets are not usable in other position than that they were installed for, which is for examination of a patient in about a horizontally lying position. Systems that are currently available for weight bearing imaging are less efficient than state-of-the-art MRI systems having superconductive magnets and are of a lower performance.

The information included in this Background section of the specification, including any references cited herein and any description or discussion thereof, is included for technical reference purposes only and is not to be regarded as subject matter by which the scope of the disclosure is to be bound.

### SUMMARY

The objective of the invention is to provide a high-performance system that enables imaging examinations in vertical position with increasing diagnostic accuracy and confidence for musculoskeletal diagnosis for pathologies that are subject to change of body posture and position.

The system is optimized for various predefined positions, from clinostatic, with other words horizontally lying position to orthostatic, with other words weight-bearing position due to a rotation of the imaging system and the patient from horizontal to vertical position.

In an aspect the invention provides a magnetic resonance imaging system, comprising:
a magnetic resonance imager comprising a superconducting magnet surrounding a bore;
a cooling system configured to cool the superconducting magnet through conduction cooling;
a frame rotatably supporting the magnetic resonance imager;
an actuator configured to move the magnetic resonance imager in a plurality of predetermined examination positions between a horizontal orientation and a vertical orientation of a main static magnetic field associated to the superconducting magnet.

The system may further comprise a patient support configured to support a patient in any of the plurality of predetermined examination positions.

A cooling system comprising a coldhead ensures the operational temperature for the superconductive magnet, wherein the coldhead is configured for thermal connection with at least one coil of the superconducting magnet at least through a cooling connector.

The cooling system may further optionally comprise a thermally conducting cooling segment in thermal connection with the coldhead and with any or all of a plurality of coils of the superconducting magnet. The thermal connection between the cooling segment and the superconducting coils, as well as between the coldhead and cooling segment or directly between the coldhead and superconducting coils, can be realized through individual or multiple cooling connectors, which preferably may be in the form of cooling straps with lugs made of material with superior thermal conductivity, such as copper. In some embodiments of the magnet structure the cooling segment may be missing and the coldhead may be thermally connected directly to any of the superconducting coils through individual or multiple cooling connectors, which preferably may be in the form of cooling straps with lugs made of material with superior thermal conductivity, such as copper.

In any of the embodiments the superconducting magnet coils may comprise MgB₂ material.

In any of the embodiments the patient support comprises a restraint system configured to provide the patient a steady position associated to the respective discrete oblique or vertical examination position while enabling weight bearing stance.

In some embodiments the system comprises at least a partially spherical cover configured for at least partially surrounding the magnet structure or the magnetic resonance imager. The cover may be made of MR-compatible materials, and it can be configured to be entirely or partially removable from the magnet structure or from the magnetic resonance imager.

In any of the embodiments the frame may comprise hollow rotation axis configured to receive at least one of: gradient cables, cooling hoses, radio-frequency (RF) cables, power supply cables and data transmission lines.

In some embodiments the plurality of predetermined examination positions may consist of discrete positions associated to the horizontal orientation and to the vertical orientation of the main static magnetic field.

In some embodiments the plurality of predetermined examination positions may comprise multiple discrete positions, including positions associated to the horizontal orientation, to the vertical orientation and at least one oblique orientation of the main static magnetic field.

The at least one oblique orientation of the main static magnetic field may be selected from the range of 15°, 30°, 45°, 60°, 75° relative to the horizontal orientation of the main static magnetic field. Multiple or all values may be selectable as oblique orientation. Other discrete values comprised in the range of 0° to 90° may be selectable and are contemplated, wherein 0° corresponds to horizontal orientation and 90° to the vertical orientation of the main static magnetic field.

Any of the embodiments of the system may comprise a control unit and a locking device, wherein the magnetic resonance imager may be coupled to the frame through at least one rotational bearing and wherein the control unit is configured to control the actuator and the locking device to move the magnetic resonance imager from one of the plurality of predetermined examination positions to another one of the plurality of predetermined examination positions. The at least one rotational bearing may comprise or may be hydrodynamic bearing, and the control unit may be configured to provide fluent movement between the plurality of discrete examination positions.

Any of the embodiments of the system may comprise shimming optimized for the plurality of predetermined examination positions. The shimming may be provided by any of passive shims, active shims and their combination.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the flow measurement system, as defined in the claims, is provided in the following written description of various embodiments of the disclosure and illustrated in the accompanying drawings.

### BRIEF DESCRIPTION OF THE DRAWINGS

Illustrative embodiments of the present disclosure will be described with reference to the accompanying drawings, of which:
Fig. 1 is a schematic illustration of a magnetic resonance imaging system according to the invention.
Fig. 2 schematically illustrates predetermined examination positions for operation of the magnetic resonance imager according to the invention.
Fig. 3. schematically illustrates an embodiment of the magnet structure.
Fig. 4. schematically illustrates a further embodiment of the magnet structure.
Fig. 5 schematically illustrates various embodiments of the magnetic resonance imager according to the invention.
Fig. 6 schematically illustrates further features of the magnetic resonance imager according to some embodiments of the invention.
Fig. 7 schematically illustrates yet further features of the magnetic resonance imager according to some embodiments of the invention.

### DETAILED DESCRIPTION

For the purpose of promoting and understanding the principles of the present disclosure, reference will now be made to the embodiments illustrated in the drawings, and specific language will be used to describe the same. It is nevertheless understood that no limitation to the scope of the disclosure is intended. Any alteration and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately. Features described in relation to a system, may be implemented in a computer implemented method and/or in a computer program product, in a corresponding manner.

Standard state-of-the-art MRI systems based on superconductive magnets cannot be used for weight bearing imaging because they have to be installed with a horizontal bore, which consequently does not allow for substantial weight bearing, with except for some of the limbs, hence only for very limited clinical use. Tilting of the system is not allowed, since the cooling of the superconducting magnet relies on liquid cryogens, which require gravity to point into a certain direction. The evaporated cryogens are condensed at a coldhead and the liquid cryogens are then collected at the bottom of the tank, in other words there is a fixed direction for the coolant circulation. Moreover, the swash of the cryogen caused by the tilting motion can lead to unwanted thermal and/or mechanical effects.

According to the invention, the MRI system based on superconducting magnet comprises a cylindrical bore and comprises conduction cooling, i.e. does not use liquids. Preferably, the material of the superconductor is MgB₂, having a much higher operating temperature (e.g., 20K) than conventionally used materials for superconductive magnets. Nevertheless, other materials are contemplated (e.g., YBCO, BSCCO, etc.) of which superconductive property can be achieved at temperature that conduction cooling can provide (e.g., >10K).

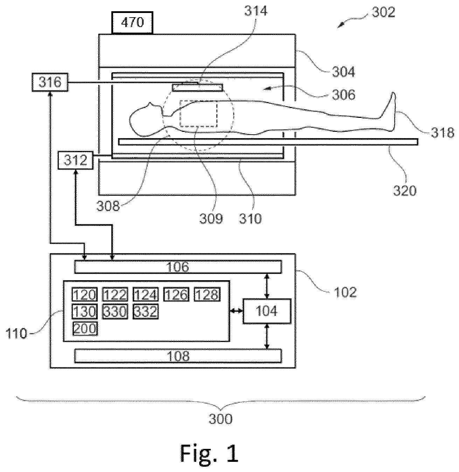

Fig. 1 illustrates an example of a system 300 based on superconducting magnet. The system 300 comprises a magnetic resonance imaging scanner or magnetic resonance imager 302, that is controlled by a computational system 102.

The magnetic resonance imaging scanner 302 comprises a magnet 304. The magnet 304 is a superconducting cylindrical-type magnet with a bore 306 through it.

Within the bore 306 of the cylindrical magnet 304 there is an imaging zone 308 where the magnetic field is strong and uniform enough for performing magnetic resonance imaging. A region of interest 309 is shown within the imaging zone 308. The magnetic resonance data is acquired typically for the region of interest. A subject 318, which may be a patient, is shown as being supported by a subject support or patient table 320 such that at least a portion of the subject 318 is movable within the imaging zone 308 and the region of interest 309.

Within bore 306 of the magnet there is also a set of magnetic field gradient coils 310, which is used for acquisition of (preliminary) magnetic resonance data to spatially encode magnetic spins within the imaging zone 308 of the magnet 304. The magnetic field gradient coils 310 are connected to a magnetic field gradient coil power supply 312. The magnetic field gradient coils 310 are intended to be representative. Typically, magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio-frequency antenna may contain multiple coil elements. The radio-frequency antenna may also be referred to as a channel or antenna. The radio-frequency coil 314 is connected to a radio-frequency transceiver 316. The radio-frequency coil 314 and radio-frequency transceiver 316 may be replaced by separate transmit and receive coils and a separate transmitter and receiver. It is understood that the radio-frequency coil 314 and the radio-frequency transceiver 316 are representative. The radio-frequency coil 314 is intended to also represent a dedicated transmit antenna and a dedicated receive antenna. Likewise, the transceiver 316 may also represent a separate transmitter and receivers. The radio-frequency coil 314 may also have multiple receive/transmit elements and the radio-frequency transceiver 316 may have multiple receive/transmit channels.

The computational system 102, in other words the control system of the MRI scanner 302, comprises one or more processors 104 or processing cores that may be in one location or may be distributed. The processor 104 is shown as being connected to an optional hardware interface 106. The hardware interface 106 may for example be used to connect the processor 102 with other components of the system 300 or with other medical devices in case necessary (e.g., ECG, vital sign camera). The hardware interface 106 may enable the processor 104 to control such components.

The transceiver 316 and the gradient controller 312 are shown as being connected to the hardware interface 106 of the computer system 102, though they may be controlled directly by the processor 104 or other processors associated to the magnetic resonance imaging system 300.

The computational system 102 is further shown as comprising a memory 110. The memory 110 is intended to represent any combination of memory or storage device which is accessible to the processor 104. The memory 110 may include volatile and non-volatile memory storage means and components. The memory 110 in some embodiments may be based on or may rely on cloud-based data stored in logical pools across disparate, commodity storage servers located on premises or in a data center managed by a third-party cloud provider. Accordingly, the memory as schematically depicted in Fig. 1 is just for illustration purposes. Therefore, any or all components comprised in memory 110 may be cloud-based.

The memory 110 is shown as containing machine-executable instructions 120. The machine-executable instructions 120 may be configured to enable the processor 104 to perform basic data processing and image processing tasks, such as reconstructing magnetic resonance images. The machine-executable instructions 120 may also in some examples contain code which enables the computational system to control other components via the optional hardware interface 106.

The memory 110 is shown as containing pulse sequence commands 330. The pulse sequence commands are commands or data that can be converted into commands, which can be used to control the magnetic resonance imager 302 to acquire k-space data 332. The memory 110 is further shown as comprising k-space data 332 that has been acquired by controlling the magnetic resonance imager 302 with the pulse sequence commands 330. The computational system 102 may also reconstruct the measured magnetic resonance imaging data 124 from the k-space data 332 and/or generate an image based on the k-space data.

The computational system 102 is further shown as comprising an optional user interface 108. The user interface 108 may for example be used by an operator to control the operation and function of the system 300. The computation system 102 could be a standalone computer system but it could also be integrated into the magnetic resonance imaging scanner or MRI system.

The memory 110 may comprise image segmentation algorithm 122. The image segmentation algorithm 122 may be a standard magnetic resonance imaging image segmentation algorithm that is configured for outputting one or more predetermined anatomical regions for a magnetic resonance imaging data. This may be for a particular field of view or anatomical region of the subject, e.g., patient.

The memory 110 may comprise the measured magnetic resonance imaging data 124. The memory 110 may further comprise an image segmentation 126 that has been received from the image segmentation algorithm 122 by inputting the measured magnetic resonance imaging data 124. The memory 110 may comprise a selected image portion 128. This is one or more regions or anatomical regions that have been identified in the image segmentation 126. The selected image portion 128 may for example be selected using a predetermined criterion that is applied to the image segmentation 126. Image segmentation is particularly beneficial for segmenting anatomical structures of the spine, for example in electrical properties tomography of spinal intervertebral discs, like disclosed in European patent application No. EP23214824 entitled "Systems and methods for electrical properties tomography" filed on December 7, 2023, and which is hereby incorporated by reference in its entirety.

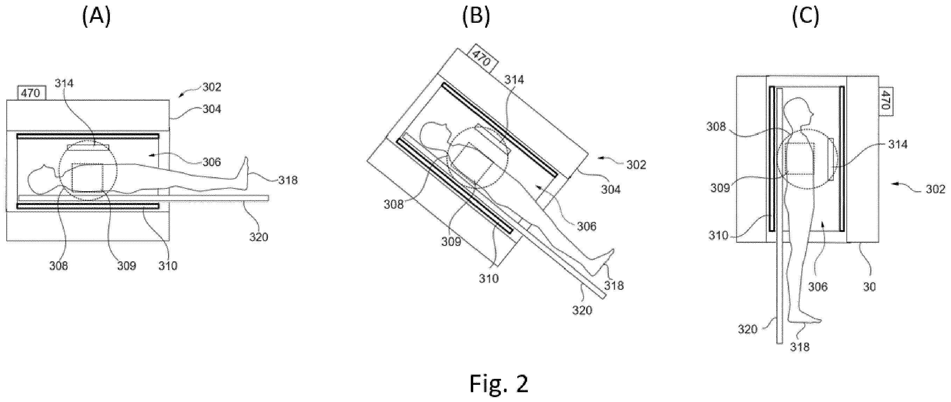

The memory may comprise a set of predetermined examination positions 200 in which the MRI scanner can be operated, which set comprises discrete positions associated to the horizontal orientation and the vertical orientation of the main static magnetic field, illustrated in Fig. 2 (A) and (C) respectively. The set may further include at least one oblique orientation of the main static magnetic field, schematically illustrated in Fig. 2 (B). In some embodiments the set may comprise at least one oblique orientation of the main static magnetic field, selectable from the range of 15°, 30°, 45°, 60°, 75° relative to the horizontal orientation of the main static magnetic field. The user interface 108 may for example be used by an operator to input one of the examination positions from the predetermined set or to select a value from a menu comprising the predetermined set.

The memory may further comprise various algorithms 130 for determining and therewith controlling shimming optimized for the plurality of predetermined examination positions. In some examples the position of passive shims is controlled by actuation, in other examples the active shims are operated according to the instructions associated to the respective predetermined examination position of the MRI scanner. In some of the examples, instead of the algorithms 130 actively determining and controlling the shimming, the shimming is controlled based on predetermined shimming schemes that have been uploaded in the memory 110 prior to examination of the subject and the shimming is automatically changed by the control system to the most optimal shimming associated to the selected one of the plurality of predetermined examination positions.

The computational system 102 or control system may control various other mechanisms or sub-systems associated with bringing the MRI scanner into the selected position for patient examination. For example, the control system may provide commands to an actuator and a locking system to move the magnetic resonance imager from one of the plurality of predetermined examination positions to a selected one of the plurality of predetermined examination positions and to lock it into position, such that the MRI scanner becomes immobile with respect to a supporting frame to which it is coupled. Accordingly, the patient support, which comprises a restraint system, is also brought into a position associated with the selected one of the plurality of predetermined examination positions and locked for providing the patient a steady position associated to the respective discrete oblique or vertical examination position while enabling weight bearing stance.

An example of conduction cooling design of MgB₂ magnets for 1.5 T and 3.0 T full body MRI systems is according to Baig et. al, Supercond Sci Technol. 2017 April; 30(4); doi: 10.1088/1361-6668/aa609b. A further example of implementing conduction cooling for MgB₂ magnets is presented in European patent application EP23197904 filed on September 18, 2023. Both references exemplifying conduction cooling of MgB₂ magnets are incorporated by reference herein in their entirety.

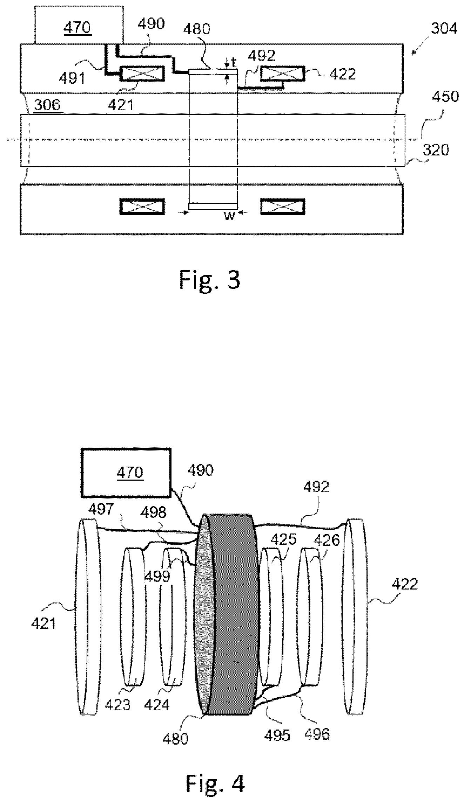

The schematic drawing in Fig. 3 illustrates an example cross-section of conduction cooled superconducting magnet 304 of a magnetic resonance imaging system 300. The magnetic resonance imager 302 includes a magnet structure 304 that surrounds a bore 306. The magnet includes magnet coils 421, 422. A longitudinal axis 450 of the bore 306 is here illustrated with a dashed line. During imaging of an object, such as a patient, the object may be placed on a patient table 320 and positioned inside the bore 306. Conduction cooling of the magnet is provided by the coldhead 470. The coldhead 470 is in this example thermally connected directly to the first magnet coil 421 via a cooling connector 491. The coldhead may also be connected to a cooling segment 480 by a cooling connector 490. The cooling segment 480 may form an arc or circle around the (longitudinal axis 450) of the bore 306. The cooling segment 480 has a width *w* and a thickness *t.* The cooling segment 480 may be thin, such as with a thickness *t* of less than 3 mm, preferably less than 2 mm and more preferably less than 1 mm. In the example, the cooling segment is thermally connected to the second magnet coil 422 with a cooling connector 492.

In some embodiments of the magnet structure 304 the cooling segment may be missing and the coldhead may be thermally connected directly to the first magnet coil 421, the second magnet coil 422 and to any optionally further magnet coils by individual cooling connectors.

Fig. 4 schematically illustrates an embodiment that is similar to the one in Fig. 3. The difference is that in this case all magnet coils 421 to 426 are thermally connected directly to the cooling segment 480 via cooling connectors 492, 495, 496, 497, 498, 499. The cooling segment 480 is thermally connected to the coldhead 470 with cooling connector 490, such that all magnet coils can be cooled.

In some embodiments wherein cooling segment is used, the system may be configured to facilitate radiation therapy according to European patent application No. EP23197904, titled "Magnetic resonance imaging radiation therapy system", filed on September 18, 2023, and which is hereby incorporated by reference in its entirety. Such system combines a magnetic resonance imager with a radiation therapy device, such as a linear accelerator. The radiation therapy device may be implemented like in any of the embodiments described in EP23197904, for example in Fig 1 of EP23197904, and the cooling segment may be provided with a larger width than the maximum width of the therapeutic beam. The cooling segment is configured such that attenuation of the therapeutic beam by the cooling segment is constant between the respective beam paths from different positions about the longitudinal axis of the bore.

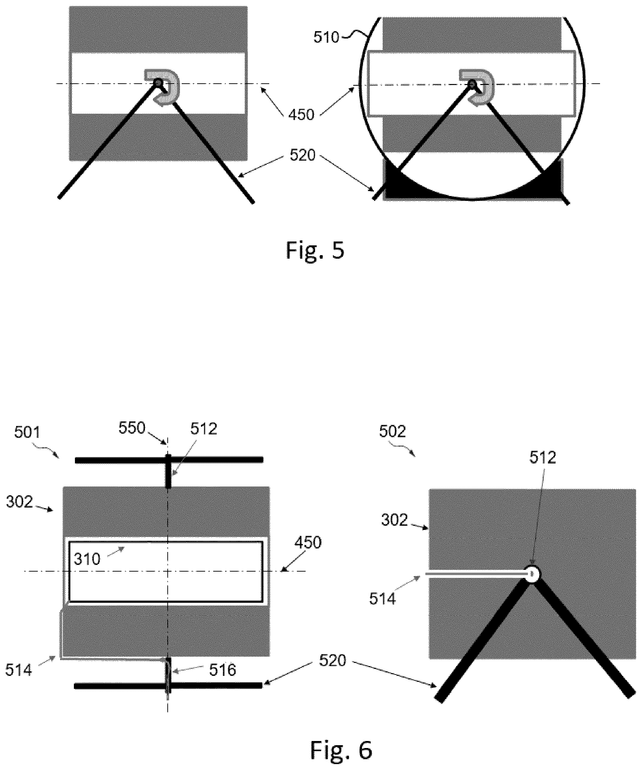

With reference to Fig. 5, a mechanical suspension is provided for the magnet structure that allows rotation of the magnet by 90° from horizontal position to vertical position, i.e. repositioning the direction of the main static magnetic field B0 from horizontal to vertical direction, with other words allowing a pitch of 90° defining a rotation around a transversal axis. A tilting frame 520 is required to be attached to the magnetic resonance imager, which facilitates rotational movement. An actuator provides the necessary movement that is transferred to the magnetic resonance scanner to perform the rotation. The actuator may be a drive unit 610 or motor that impinges continuous movement which is coupled via a mechanism to the magnet structure or magnetic resonance imager. Alternatively, a stepper motor may be used, with the benefit of high precision due to providing discrete and highly reproducible steps. The actuator may be integrated within the tilting frame or may be a standalone unit. Rotational movement of the magnetic resonance imager with respect to the tilting frame may be controlled by control logic, including means for pinch protection. Optionally, a cover 510 may be provided for the magnetic resonance imager, which may have a partial or complete spherical shape, such that any risk of pinching during the rotation is minimized. In an embodiment the actuator may be a lever that is actuated manually by a user of the magnetic resonance imaging system.

All mechanical connections inside the magnetic resonance imager (e.g., gradient coil to magnet structure) need to be reinforced, such that they also work properly without substantial loss of imaging quality for a vertical orientation of the main static magnetic field upon tilting the magnetic resonance imager 90°. In particular, flanges may be installed, which prevent a relative motion of different components (gradient coil, RF coil) in axial direction. Due to the practical size of the cylindrical magnet (< 2 m length) the tilting motion itself does not pose severe constraints on the room height.

Fig. 6 illustrates schematically a top-view cross section 501 and a side-view 502 of a magnetic resonance imager 302 exemplary embodiment. All connections to the magnet (cables, hoses, etc.) have to be made close to the axis of rotation 550, where only a twist of maximum 90° is expected, which does not pose a major problem for the corresponding connections. The cabling to the gradient coil 310, which is typically connected at its ends, can either be made by cables 516 and/or busbars 514. In any case, the corresponding forward and return conductors have to be routed in close vicinity and in a mechanically connected way, such that the net force on the conductors in the magnetic field is minimized. Moreover, the cables have to be routed as parallel as possible to the magnetic field lines of the main magnet, which reduces the magnetic forces, too. At least "diagonal" or circumferential cables/busbars on the outer surface of the magnet have to be avoided. Near or at the rotating axis 550, cables have to be used to allow for the twist required for the tilting. In a preferred embodiment, the rotating axis 512 is hollow and all the necessary cables, optical fibers and tubes (e.g., gradient cables, cooling hoses, RF cables, power supply cables and data transmission lines) can be fed through it. This minimizes the risk of damaging the connections. If the tilting requires additional flexibility of the cables, then drag chains can be installed.

The mechanical suspension is made in a way to allow the desired tilt while at the same time to withstand the stress of the potentially heavy vibrating magnet (vibration induced by gradient and B0 field interaction): the stationary part of the system is a solid frame 520, featuring the same bearings 602 as conventional magnets (basically foam pads) to avoid acoustic noise coupling from magnet chassis to the building. The rotary bearing 602 to allow tilting of the magnet has two operating states. In the first one the magnet can be softly rotated in a patient comfortable manner and in the second one the rotary movement is prevented by means of a clamp device (e.g., like known form clutches of cars).

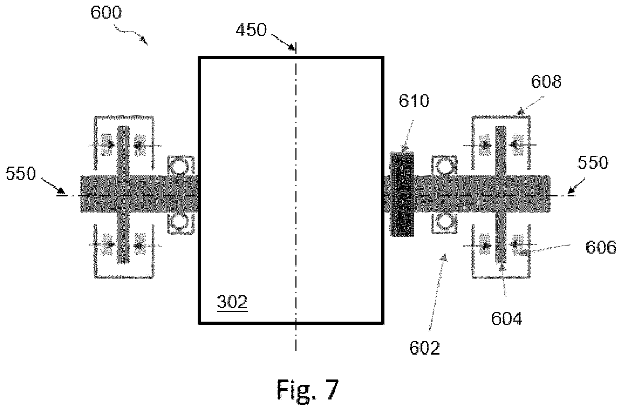

The design of that clamp device 608, or in other words the locking device, may be made in a way that in the second operational state repeated vibrational stress caused by the magnet-gradient coil interaction does not harm the bearing. An example is shown in Fig. 7, wherein for the first operational state the clutch is open, allowing for controlled rotation, and for the second operational state the clutch is in closed position, which assures mechanical fixation, preventing dynamic load on the ball bearing. The clamp device 608, which in the example is a clutch/disk-break assembly, comprises the clutch disk 604 and clutch pads 606. The operation of the motorized drive 610 of the rotation with coupling and decoupling can be automatized and controlled by a standalone control unit and/or by the computational system 102. Additionally or alternatively, coupling and decoupling of the motorized drive of the rotation may be effected manually by a manual drive comprising for example a wheel and lever mechanism.

The rotary or rotational bearing 602 may comprise hydrodynamic bearing. The control unit is configured to provide fluent movement between the plurality of discrete examination positions.

With reference to any of the embodiments, the mechanical suspension may be with respect to the floor of the examination room or with respect to the ceiling. The tilting frame 520 attached to the magnetic resonance imager may therefore accordingly be attached either to the floor or to the ceiling.

All magnets in the field need to be shimmed. This is a method to compensate for inhomogeneity of the static field induced by site-specific ferromagnetic parts in the surrounding of the magnet. Compensation is done by adding a number of ferromagnetic plates at dedicated positions close to the magnet to reach the desired homogeneity. The shimming procedure is as follows, (a) determining the magnetic field homogeneity, (b) calculating a distribution of ferromagnetic plates that improves homogeneity, (c) adding plates and determining homogeneity again, with (d) iterating until the desired homogeneity is reached. The iteration is required due to the non-linear behavior of the plates. For the tiltable or rotatable magnet it is proposed to consider the rotation and determine the inhomogeneity for different predetermined rotation angles (e.g., 15°, 30°, 45°, 60°, 75°). The algorithm determining the plate distribution is extended by considering several angles to reach a compromise distribution so that an acceptable magnetic field homogeneity is reached for all predetermined angles at which imaging is desired and is performed, e.g., B0 magnetic field inhomogeneity ≤ 1 ppm rms for 40-cm diameter of spherical volume (DSV). This can be a linear programming algorithm that has as variables the shim iron stack heights, as cost function the shim iron volume and the target field tolerances as boundary conditions. The predetermined tilt angles for imaging might be only a limited number of angles, such as for example only 0° and 90°, in other words in horizontal and in vertical position. Additionally, other oblique configurations, for example any or all of the following angles 15°, 30°, 45°, 60°, 75° may be added to cover the predetermined angles for practical examination of the patient. In addition to the passive shims discussed hereinabove, active shims, i.e. actively driven coils might be employed to further enhance homogeneity, achieving B0 magnetic field inhomogeneity below 0.45 ppm rms for 40-cm DSV. Using only active shims, i.e. currents directed through specialized coils to generate a "corrective" magnetic field, associated to any of the predetermined examination positions between a horizontal orientation and a vertical orientation of the main static magnetic field, is also contemplated. In some embodiments adjusting active shims according to the selected one of the predetermined examination position is provided, besides passive shims at fixed locations.

Modifications of the patient support is required for allowing examination of the patient in any of the oblique or vertical positions, such that the patient is sufficiently restrained to remain in stable position associated to the respective discrete oblique or vertical examination position while enabling weight bearing stance. Especially, a stand and/or plate has to be provided on the table, such that the patient can stand on own feet when the magnet axis is in vertical position. Moreover, the support mechanism of the table has to be modified such that the table cannot lift off. This locking mechanism can e.g., be realized by guiding the wheels of the table with C-shaped rails or by an additional hook. The same mechanism also has to provide sufficient strength to hold the table in position when it is tilted, i.e. to prevent the table slipping out. The use of gearwheels and cograils is an option in this context.

The logical operations making up the embodiments of the technology described herein are referred to variously as operations, steps, objects, elements, components, or modules. Furthermore, it should be understood that these may be arranged or performed in any order, unless explicitly claimed otherwise or a specific order is inherently necessitated by the claim language. It should further be understood that the described technology may be employed in single-use and multi-use electrical and electronic devices for medical or nonmedical use.

Any combination of features discussed with reference to Figs. 5 to 7 are contemplated. Additionally or alternatively, further combination with features discussed with respect to any of the magnet structures is also contemplated. Moreover, additionally or alternatively, further combination with features discussed with reference to Fig. 1 are contemplated.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of. Connection references, e.g., attached, coupled, connected, and joined are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary embodiments of the flow measurement system as defined in the claims. Although various embodiments of the claimed subject matter have been described above with a certain degree of particularity, or with reference to one or more individual embodiments, those skilled in the art could make numerous alterations to the disclosed embodiments without departing from the spirit or scope of the claimed subject matter.

Still other embodiments are contemplated. It is intended that all matter contained in the above description and shown in the accompanying drawings shall be interpreted as illustrative only of particular embodiments and not limiting. Changes in detail or structure may be made without departing from the basic elements of the subject matter as defined in the following claims.

## Claims

1. A magnetic resonance imaging system, comprising:
a magnetic resonance imager (302) comprising a superconducting magnet (304) surrounding a bore (306);
a cooling system configured to cool the superconducting magnet through conduction cooling;
a frame (520) rotatably supporting the magnetic resonance imager;
an actuator configured to move the magnetic resonance imager in a plurality of predetermined examination positions between a horizontal orientation and a vertical orientation of a main static magnetic field associated to the superconducting magnet.

2. The system of claim 1, further comprising a patient support (320) configured to support a patient in any of the plurality of predetermined examination positions.

3. The system of claim 1 or 2, wherein the cooling system comprises a coldhead (470) in thermal connection with at least one coil (491) of the superconducting magnet at least through a cooling connector (491).

4. The system of claim 3, wherein the cooling system further comprises a thermally conducting cooling segment (480) in thermal connection with the coldhead and a plurality of coils of the superconducting magnet.

5. The system of claim 3 or 4, wherein the coils comprise MgB₂ material.

6. The system of any of the preceding claims, wherein the plurality of predetermined examination positions consists of discrete positions associated to the horizontal orientation and the vertical orientation of the main static magnetic field.

7. The system of any of the claims 1 to 5, wherein the plurality of predetermined examination positions comprises multiple discrete positions, including positions associated to the horizontal orientation, to the vertical orientation and to at least one oblique orientation of the main static magnetic field.

8. The system of claim 7, wherein the at least one oblique orientation of the main static magnetic field is any or all of 15°, 30°, 45°, 60°, 75° relative to the horizontal orientation of the main static magnetic field.

9. The system of any of the preceding claims, further comprising a control unit (102) and a locking device (608), wherein the magnetic resonance imager is coupled to the frame through at least one rotational bearing (602) and wherein the control unit is configured to control the actuator and the locking device to move the magnetic resonance imager from one of the plurality of predetermined examination positions to another one of the plurality of predetermined examination positions.

10. The system of claim 9, wherein the at least one rotational bearing comprises hydrodynamic bearing and wherein the control unit is configured to provide fluent movement between the plurality of discrete examination positions.

11. The system of any of the preceding claims, further comprising shimming optimized for the plurality of predetermined examination positions.

12. The system of claim 11, wherein the shimming is provided by any of passive shims, active shims and their combination.

13. The system of any of the preceding claims, wherein the patient support comprises a restraint system configured to provide the patient a steady position associated to the respective discrete oblique or vertical examination position while enabling weight bearing stance.

14. The system of any of the preceding claims, further comprising at least a partially spherical cover configured for at least partially surrounding the magnetic resonance imager.

15. The system of any of the preceding claims wherein the frame comprises hollow rotation axis configured to receive at least one of: gradient cables, cooling hoses, RF cables, power supply cables and data transmission lines.
